# EUROPÄISCHE PATENTSCHRIFT

(11) **EP 0 628 294 B1**
(45) Veröffentlichungstag und Bekanntmachung des Hinweises auf die Patenterteilung: **13.08.1997**
(21) Anmeldenummer: 94108417.0
(22) Anmeldetag: 01.06.1994
(51) Int. Cl.: A61F 2/34, A61F 2/30

(54) **Beckenteilendoprothese**
Partial pelvic endoprothesis
Endoprothèse partielle du bassin

(30) Priorität: 08.06.1993 DE 4319010
(43) Veröffentlichungstag der Anmeldung: 14.12.1994
(73) Patentinhaber: ESKA Implants GmbH & Co., 23556 Lübeck (DE)
(72) Erfinder: Grundei, Hans, Dr., D-23558 Lübeck (DE); Gradinger, Reiner, Prof. Dr., D-23562 Lübeck (DE)
(74) Vertreter: Fuchs, Luderschmidt & Partner Patentanwälte

(56) Entgegenhaltungen:
- DE-A- 2 605 180
- DE-A- 3 629 799
- DE-A- 3 710 233
- DE-C- 4 133 433
- DE-U- 9 111 221
- GB-A- 2 016 275

## Beschreibung

Die Erfindung betrifft eine Beckenteilendoprothese zum Ersatz eines Beckenteils im Bereich des Hüftgelenks mit einem Grundkörper, der eine kalottenförmige Aussparung zur Aufnahme einer künstlichen Hüftgelenkspfanne aufweist, und einem ersten Anschlußelement zur Herstellung einer Verbindung des Grundkörpers mit dem Darmbeinbereich des Restbeckens.

Eine Endoprothese mit diesen Merkmalen als Teilmerkmale ist bekannt aus der DE 36 29 799 A1. Darin ist beschrieben, daß zwei Anschlußelemente durch Zapfen mit entsprechenden Aufnahmen am Grundkörper verbunden sind, wobei das eine Anschlußelement - wie erwähnt - zur Herstellung der Verbindung des Grundkörpers mit dem Darmbeinbereich des Restbeckens, das andere Anschlußelement hingegen zur Herstellung der Verbindung des Grundkörpers mit dem Schambeinbereich des Restbeckens dient. Die eigentliche Anbringung der Anschlußelemente an den Restknochen erfolgt durch an den Knochenteilen zum Anliegen kommende Plattenteile mit Bohrungen, durch die Knochenschrauben quer durch den Restknochen geschraubt werden.

Zwar gestatten neuere Diagnostizierverfahren die Erfassung von Tumoren durch Computertomographie und die Nachbildung der Knochenbereiche sowie der von Tumor befallenen Bereiche in Kunststoff. Anhand dieser Modelle können die Operateure dann vor der eigentlichen Operation festlegen, wie das Implantat letztendlich auszusehen hat, welche Resektionen vorher durchgeführt werden müssen und wie einzelne Handgriffe während der Operation auszusehen haben. Diese vorbereitenden Maßnahmen sind zwar sehr hilfreich, garantieren aber keinen reibungslosen Operationsverlauf. Besonders nachteilig bei der bekannten Endoprothese wird dabei empfunden, daß diese zu unflexibel ist. So kann es während des massiven Eingriffs, währenddessen Teile des ganzen Beckens, einschließlich der natürlichen Gelenkpfanne, reseziert werden, durchaus dazu kommen, daß die Knochenschrauben an zunächst vermeintlich richtigen Stellen in den Knochen geschraubt werden, während es sich nach dem Zusammenbau der modularen Prothese herausstellen kann, daß eine Positionskorrektur vonnöten ist. In diesem Falle müssen also die Knochenschrauben zumindest teilweise wieder entfernt werden, das eine oder das andere Anschlußelement in anderer Position wieder eingesetzt werden und in den ohnehin durch die massive Resektion geschwächten Knochen erneut Knochenschrauben in die Knochen geschraubt werden unter Belassung der dann nicht mehr benötigten und von dem erstmaligen Versuch herrührenden Durchgänge durch den Knochen. Darüber hinaus ist zwar ein Verschwenken des Grundkörpers um den einen Zapfen des ersten Anschlußelementes möglich, bevor der sogenannte Festsitz zwischen dem ersten Anschlußelement und dem Grundkörper hergestellt wird. Wird allerdings der Grundkörper bei der bekannten Endoprothese um die Zapfenachse verschwenkt, so wird der Grundkörper aus der anatomisch vorgegebenen Ebene einer natürlichen Hüftgelenkspfanne herausgedreht, so daß unter Umständen das künstliche Hüftgelenk auf einer anderen Höhe zu liegen kommt als das natürliche Hüftgelenk auf der anderen Seite des Beckens, welches im Patientenkörper belassen wird. Die Folge hiervon sind mögliche Belastungen des Restknochens, woraus sich erhebliche postoperative Komplikationen ergeben können.

Eine weitere Endoprothese zum Ersatz eines menschlichen Beckenteils ist bekannt geworden aus DE-41 33 433 C1. Gegenüber jener aus der vorerwähnten Druckschrift verfügt die darin beschriebene Endoprothese über einen Freiheitsgrad mehr in der Verstellbarkeit. Sie besteht aus einer Außenschale, deren Wandung durch eine Vielzahl von Durchbohrungen durchsetzt ist. Durch diese Durchbohrung wird eine Schraube gesetzt, um mit einem konischen Zapfen verschraubt zu werden, welcher an einem Anschlußelement für den Darmbeinstumpf angeformt ist. Ein Zwischenstück zwischen dem Zapfen und der Außenschale wirkt wie ein Abstandshalter. Die Auswahl der Durchbohrung, durch welche die Schraube gesetzt wird, soll intraoperativ vom Operateur vorgenommen werden, damit eine vertretbare Stellung der Außenschale in bezug auf die übrigen Beckenteile eingestellt werden können soll. Danach wird die Außenschale über weitere Verbindungsteile mit dem Schambeinstumpf verbunden.

Einmal davon abgesehen, daß die metallische Außenschale durch die Vielzahl der Durchbohrungen mechanisch erheblich geschwächt ist, wird es als nachteilig angesehen, daß der Operateur während des Eingriffs mit einer Vielzahl von Einzelteilen hantieren muß, bis die Endoprothese eine vertretbare Stellung erhält. Diese Vielzahl von Elementen müssen bei jeder Änderung der Einbaulage versetzt werden.

Vor diesem aufgezeigten Hintergrund ist es nun das Ziel der vorliegenden Erfindung, eine Beckenteilendoprothese anzugeben, die aus einem Set von Elementen erstellbar ist, bei der einzelne Elemente auch noch intraoperativ ausgetauscht werden können und deren Einbaulage während der Implantation problemlos eingestellt werden kann.

Gelöst wird dieses Problem durch die Beckenteilendoprothese gemäß dem Anspruch 1. Weitere vorteilhafte Weiterbildungen sind in den Unteransprüchen angegeben.

Die erfindungsgemäße Beckenteilendoprothese wird demgemäß zumindest aus zwei Teilen oder Elementen aus dem Set erstellt, nämlich aus einem Grundkörper, der eine kalottenförmige Aussparung zur Aufnahme einer künstlichen Hüftgelenkspfanne sowie einen Ansatz aufweist, in welchem mindestens eine Aufnahmebohrung ausgebildet ist, deren Längsachse mit der Polachse der Aussparung einen Winkel β im Bereich von 20°< β <30°, vorzugsweise β = 25°, einschließt, sowie aus einem ersten Anschlußelement zur Herstellung einer Verbindung des eben beschriebenen Grundkörpers mit dem Darmbeinbereich des Restbeckens, wobei die Verbindung über einen selbsthemmenden Sitz eines Zapfens des ersten Anschlußelementes in der Aufnahmebohrung im Ansatz des Grundkörpers herstellbar ist, wobei der Zapfen von dorsal nach ventral gesehen in einem Winkel γ im Bereich 25°< γ <45°, vorzugsweise γ = 35°, und von medial nach lateral in einem Winkel δ im Bereich 0< δ <10°, vorzugsweise δ= 5°, geneigt ist. Gegebenenfalls können am erwähnten Zapfen Rotationssicherungsstifte vorgesehen sein, die in entsprechende Bohrungen im Ansatz des Grundkörpers greifen. Eine zusätzliche Sicherungsschraube kann vorgesehen sein, um den Sitz des Zapfens in der Aufnahmebohrung dauerhaft und unverrückbar zu machen.

Der Angabe der vorerwähnten Winkelbereiche liegt die folgende Erkenntnis zugrunde: Selbstverständlich variieren die absoluten Maße eines Beckens von Patient zu Patient. Was jedoch erstaunlicherweise nicht in weiten Grenzen variiert, sind die relativen Maße und Winkellagen. Dies bedeutet also, daß sich die gleichen Winkellagen beispielsweise zwischen Schambein und Darmbein im Becken der unterschiedlichsten Größe wiederfinden. Diese Erkenntnis gestattet es erst, eine Beckenteilendoprothese, wie beschrieben nach standardisierten Vorgaben zu fertigen.

Das Minimalimplantat besteht also aus zwei Elementen, dem Grundelement und einem Anschlußelement zum Darmbeinstumpf. Das Set zur Erstellung Endoprothese gestattet aber darüber hinaus die Erstellung weiterer Ausführungsformen der Endoprothese, wie nachfolgend erläutert werden wird.

Gemäß einer vorteilhaften Weiterbildung weist der Ansatz des Grundkörpers nicht nur eine, sondern zwei Aufnahmebohrungen auf. Deren Winkelbeziehung ist so, daß ihre Längsachsen einen Winkel α im Bereich von 40°< α <60°, vorzugsweise α = 50°, miteinander und jeweils einen Winkel β im Bereich von 20°< β <30°, vorzugsweise β = 25°, mit der Polachse der Aussparung einschließen.

Diese Ausbildung gestattet zum einen, daß der Grundkörper sowohl für die rechte, als auch für die linke Beckenseite verwendet werden kann, ohne Einschränkung der Einstellungsmöglichkeiten während der Operation. Darüber hinaus gestattet die Ausbildung zweier Aufnahmebohrungen den Anschluß eines weiteren Anschlußelementes, wie nachstehend beschrieben wird.

Gemäß einer weiteren vorteilhaften Ausführungsform wird dem Minimalimplantat aus dem Grundkörper mit zwei Aufnahmebohrungen und dem ersten Anschlußelement ein weiteres, zweites Anschlußelement zugeordnet zur Herstellung einer Verbindung des Grundkörpers mit dem Schambeinbereich des Restbeckens. Die Verbindung ist über einen selbsthemmenden Sitz eines zapfenförmigen Verbindungselementes in eine der Aufnahmebohrungem im Ansatz des Grundkörpers herstellbar.

Vorzugsweise weisen die beiden Aufnahmebohrungen gleiche Größe und gleiche Form auf und entsprechen die Außenkonturen des zapfenförmigen Verbindungselementes des zweiten Anschlußelementes jenen des Zapfen des ersten Anschlußelementes. Diese Weiterbildung ermöglicht die Verwendung des Grundkörpers im rechten und im linken Beckenbereich, sowie die Verwendbarkeit des zweiten Anschlußelementes ebenfalls sowohl rechts als auch links.

Vorteilhaft sind die Aufnahmebohrungen als konische Klemmhülsen und der Zapfen und das zapfenförmige Verbindungselement als konische Steckzapfen ausgebildet.

Vorteilhaft ist es, wenn die Aufnahmebohrung bzw. Aufnahmebohrungen im Ansatz bis in die Innenkalotte des Grundkörpers reichen. Damit sind die Aufnahmebohrungen auch von der Innenseite des Grundkörpers zugänglich, um beispielsweise eine Verschraubung des zapfenförmigen Elementes des zweiten Anschlußelementes und/oder des Zapfens des ersten Anschlußelementes vornehmen zu können.

Die vorstehend beschriebenen Weiterbildungen bezogen sich auf den Grundkörper. Aber auch für sowohl das erste als auch für das zweite Anschlußelement werden vorteilhafte Weiterbildungen nachstehend angegeben.

Gemäß einer ersten Ausführungsform weist das erste Anschlußelement eine Basis zur Anlage an dem Darmbeinbereich auf. Der Darmbeinbereich besteht nach der Resektion aus dem Darmbeinstumpf. Mittels einer speziellen Fräslehre lassen sich so die normierten und standardisierten Resektionen durchführen. Von der Basis ragen lateral zwei Anschlagslaschen und medial eine Anschlagslasche ab, zwischen denen der Darmbeinstumpf zu liegen kommt. Es ist ein entlang der Basis verfahrbares und dieses durchsetzendes, in einer im Restbecken intramedullär anzubringenden Nut zu setzendes Stielteil vorgesehen, an welchem in Richtung auf den Grundkörper der Zapfen angeformt ist, welcher in eine der Aufnahmebohrungen im Grundkörper einzusetzen ist. Operativ geht man also nach der Resektion des Darmbeinbereiches so vor, daß eine intramedulläre Nut im Darmbeinknochen gefräst wird, in welche das erwähnte Stielteil eingesetzt wird. Eine Besonderheit ist, daß dieses Stielteil entlang der Basis des Anschlußelementes verfahrbar ist mittels einer mechanischen Verstelleinheit. Die Verstelleinheit kann beispielsweise durch eine Mechanik mit einer Gewindespindel realisiert sein. Das Stielteil durchsetzt die Basis, indem in der Basis ein Langloch vorgesehen ist. Diese Verfahrbarkeit bietet einen hinreichenden Freiheitsgrad für die intraoperative Einstellbarkeit der Endoprothese.

Vorzugsweise ist das Stielteil in Höhe des ersten Anschlußelementes von der Achse des Zapfens um 5 bis 20 mm nach dorsal versetzt angeordnet. Dies ist im Hinblick darauf vorgesehen, als daß in dem Darmbeinstumpf dann eine intramedulläre Nut an solcher Stelle eingebracht werden kann, wo genügend Spongiosa im Knochen durch Ausfräsung entfernt werden kann und genügend Raum erzeugt werden kann, damit das Stielteil überhaupt intramedullär eingesetzt werden kann.

Wenn auch ein zweites Anschlußelement für die Verbindung des Grundkörpers mit dem Schambeinstumpf zur Erstellung des Implantates vorgesehen ist, sind zwei verschiedene Ausführungsformen hierfür vorgesehen.

Beiden Ausführungsformen ist gemeinsam, daß das zweite Anschlußelement ein schambeinseitiges Anschlußstück mit einer am Schambeinstumpf befestigbaren Lasche und einem Kugelkäfig sowie ein Überbrückungselement aufweist, dessen eines Ende als im Kugelkäfig gelagerte Kugel ausgebildet und dessen anderes Ende mit dem zapfenförmigen Verbindungselement verbindbar ist.

Beiden Ausführungsformen des zweiten Anschlußelementes ist auch gemeinsam, daß das jeweilige Überbrückungselement durch eine der jeweiligen Nuten in das zapfenförmige Verbindungselement greift und dort mit ihm verschraubt ist.

Die erste Ausführungsform des Überbrückungselementes besteht aus einer Gewindehülse, die an ihrem dem Grundkörper zugewandten Ende eine weitere Kugel trägt, die in einen Kugelkäfig greift, an dem rückseitig ein Fixationsstab befestigt ist, der mit dem zapfenförmigen Verbindungselement verschraubbar ist.

Dieses Überbrückungselement weist also bildlich gesprochen etwa die Form einer Hantel auf.

Bei der zweiten Ausführungsform des zweiten Anschlußelementes besteht das Überbrückungselement aus einem Drahtseil, welches im Inneren des zapfenförmigen Verbindungselementes verschraubbar ist.

Das zuerst erwähnte, hantelförmige Überbrückungselement dient einem festeren Schluß zwischen dem Grundkörper und dem Schambeinstumpf. Das Überbrückungselement in Form eines Drahtseiles ist etwas weicher. Es kann nur Zugkräfte übertragen, die aber ohnehin im Becken über alle Maßen überwiegen, also Druckkräfte auf das Drahtseil praktisch nicht auftreten.

Alle Kugeln der Überbrückungselemente werden in einer Art Lagerkäfig gehalten, die vorzugsweise mit einer Lagerschale, beispielsweise aus hochverdichtetem Polyethylen oder anderen geeigneten Gleitpartnern bestehen. Hierdurch wird ein Metallabrieb verhindert.

Die spezielle Ausbildung der Überbrückungselemente dient dazu, Mikrobewegungen der Endoprothesenteile gegenüber dem Restbecken zu ermöglichen. Im Falle beispielsweise der Endoprothese gemäß der bereits genannten DE 36 29 799 C2 überbrückt das dort völlig starre Implantat das Darmbein und das Schambein. Aufgrund der völlig unterschiedlichen Elastizitätsmodulen des Metalls der Endoprothese einerseits und des im Körper verbleibenden Restknochens wird dieser aufgrund der starren Überleitung der Kräfte von seiten des Implantats überstrapaziert. Dies soll durch die erfindungsgemäße Ausgestaltung vermieden werden.

Die Erfindung wird nun anhand einiger Ausführungsbeispiele gemäß der Zeichnungen näher erläutert. Hierbei zeigt:
- **Figur 1**: eine Beckenteilendoprothese aus Grundkörper und erstem Anschlußelement im implantierten Zustand am Darmbeinstumpf,
- **Figur 2**: eine Ausführungsform für den Grundkörper,
- **Figur 3**: den Grundkörper, gesehen in Richtung des Pfeiles III in Figur 2,
- **Figur 4**: den Grundkörper, gesehen in Richtung des Pfeiles IV in Figur 3,
- **Figur 5**: eine Schnittansicht des Grundkörpers,
- **Figur 6**: eine Ausführungsform des ersten Anschlußelementes,
- **Figur 7**: das erste Anschlußelement, gesehen in Richtung des Pfeiles VII in Figur 6,
- **Figur 8**: das erste Anschlußelement, gesehen in Richtung des Pfeiles VIII in Figur 6,
- **Figur 9**: eine Schnittansicht durch den Grundkörper mit daran montiertem zweiten Anschlußelement gemäß einer Ausführungsform,
- **Figur 10**: eine Schnittansicht des Grundkörpers mit einem Überbrückungselement eines zweiten Anschlußelementes gemäß einer anderen Ausführungsform, und
- **Figur 11**: eine Beckenteilendoprothese mit Grundkörper und zwei Anschlußelementen, ventral gesehen.

In den Zeichnungsfiguren sind dieselben Teile mit den gleichen Bezugszeichen versehen.

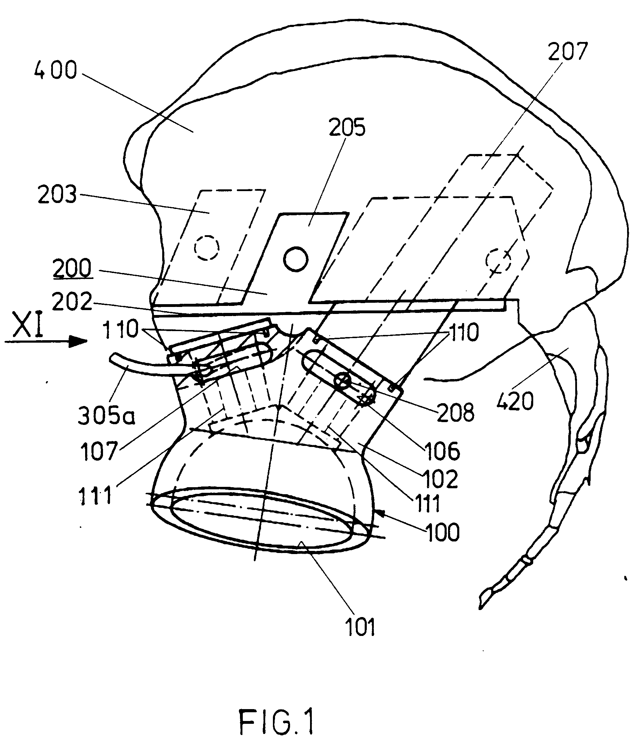

Einen ersten Überblick verschafft Figur 1. Diese zeigt ein Restbecken mit dem Darmbeinstumpf 400 und Kreuzbein 420.

Am Darmbeinstumpf befestigt ist das erste Anschlußelement 200, welches mit seinen Anschlagslaschen 203, 205 den Darmbeinstumpf einfassen. Angedeutet ist das Stielteil 207, das intramedullär in eine in den Darmbeinstumpf 400 gefräste Nut eingreift. Das Stielteil durchsetzt in weiter unten näher beschriebener Weise die Basis des ersten Anschlußelementes 200. Auf der anderen Seite der Basis ist an dem Stielteil 207 ein Zapfen 201 angeformt, der als Verbindungselement zum Grundkörper 100 der Endoprothese dient. Der Zapfen ist - wie schon aus Figur 1 ersichtlich - von dorsal nach ventral geneigt in einem Winkelbereich von 25° bis 45°. Hierauf wird weiter unten näher eingegangen.

Der dargestellte Grundkörper 100 verfügt über eine kalottenförmige Aussparung 101, welche zur Aufnahme einer künstlichen Hüftgelenkspfanne dient. Der Grundkörper trägt einen Ansatz 102, in dem im abgebildeten Ausführungsbeispiel zwei Aufnahmebohrungen 103 und 104 ausgebildet sind. Der linke Bereich des Ansatzes 102 ist mit angedeuteten Strichlinien versehen, um zu veranschaulichen, wie sich eine andere Ausführungsform darstellt, bei der die linke Aufnahmebohrung nicht vorhanden zu sein braucht.

In der linken Aufnähmebohrung greift vorliegend jedoch ein Überbrückungselement 305a einer Ausführungsform eines zweiten Anschlußelementes.

Angedeutet sind noch Bohrungen 110 in der Peripherie der Bohrungswandungen, die als Rotationssicherungsbohrungen vorgesehen sind. In diese greifen Sicherungsstifte ein, die am Zapfen des ersten Anschlußelementes entsprechend angeordnet sind. Dies stellt eine zusätzliche Rotationssicherung dar. Außen am Ansatz 102 des Grundkörpers sind beiderseitig beider Aufnahmebohrungen jeweils zwei tangential verlaufende Nuten 106, 107 vorgesehen, welche die Aufnahmebohrungen bereichsweise tangential durchbrechen. Der Zweck dieser Nuten ist, daß das Überbrückungselement 305a durch die Bohrungswandung hindurch zu einem zapfenförmigen Verbindungselement eines zweiten Anschlußelementes greifen kann.

Im Falle des ersten Anschlußelementes 200 ist ein Sicherungsstift 208 im Ansatzbereich des Zapfens vorgesehen, der bei Herstellung des selbsthemmenden Sitzes des Zapfens in der Aufnahmebohrung durch Federkraft in die Nut 106 gedrückt wird.

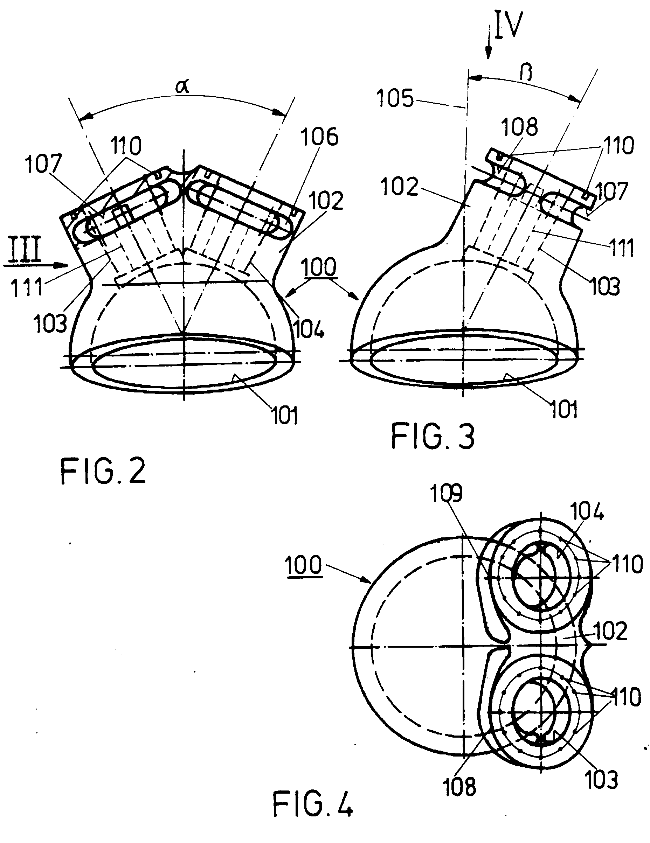

Figur 2 zeigt den Grundkörper 100 mit dem Ansatz 102 mit zwei Aufnahmebohrungen. Die Aufnahmebohrungen sind so angeordnet, daß sie zwischen sich einen Winkel α einschließen, der im Bereich von 40° bis 60° liegt und vorzugsweise 50° beträgt. In Verbindung mit Figur 3 wird deutlich, daß beide Bohrungslängsachsen mit der Polachse 105 einen Winkel β einschließen, der im Bereich von 20° bis 30° liegt und vorzugsweise 25° beträgt. Figur 3 ist auch gültig für den Fall, daß im Ansatz 102 lediglich eine Aufnahmebohrung vorgesehen ist. Angedeutet in Figuren 2 und 3 ist noch ein Gewindegang 111 im Zapfen eines Anschlußelementes, in den eine Schraube schraubbar ist zur zusätzlichen Sicherung gegen ein sich Lösen der selbsthemmenden Verbindung. Deutlich wird auch noch einmal die beiderseitige Anordnung von Tangentialnuten 106 bis 109 aus den Figuren 2 bis 4. Aus Figur 4 wird im übrigen besonders deutlich die konzentrische Anordnung der Sicherungsbohrungen um die Öffnungen der Aufnahmebohrungen 103 und 104.

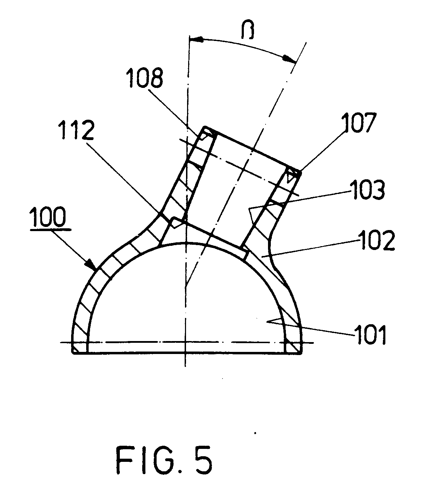

Figur 5 zeigt die Schnittansicht des Grundkörpers 100 mit den schon beschriebenen Elementen. Aus Figur 5 wird jedoch besonders deutlich, daß die Aufnahmebohrung 103 bis in die Innenkalotte 101 des Grundkörpers 100 reichen. In dem inneren Bereich ist eine Ringschulter 112 vorgesehen, gegen die sich ein Schraubenkopf legen kann, wenn sich beispielsweise der Zapfen 201 des ersten Anschlußelementes 200 mit einem Innengewinde 211 in der Aufnahmebohrung 103 befindet und die Schraube mit dem Innengewinde 211 verschraubt wird.

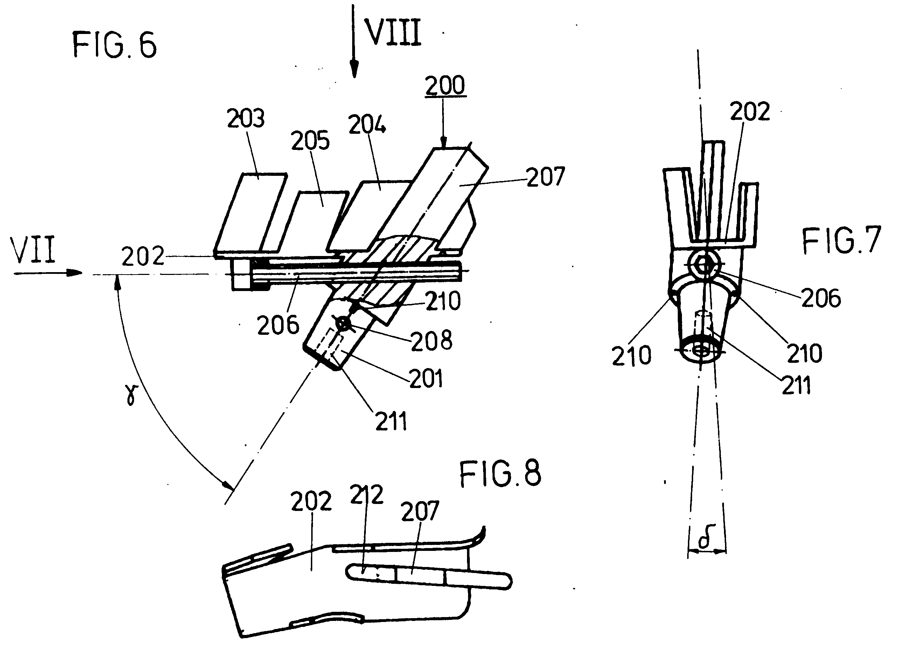

Figur 6 zeigt die Seitenansicht einer Ausführungsform des ersten Anschlußelementes 200, also jenes, welches die Verbindung vom Darmbeinstumpf 410 bis zum Grundkörper 100 herstellt.

Es weist eine plattenförmige Basis 202 auf, von der lateral zwei Anschlagslaschen 203 und 204 und medial eine Anschlagslasche 205 nach oben abragt. Der Darmbeinstumpf kommt auf der plattenförmigen Basis 202 zu liegen und wird dann von den Anschlagslaschen 203, 204 und 205 eingefaßt. Voraussetzung hierfür allerdings ist, daß eine Einfräsung in das spongiöse Material des Restdarmbeins eingebracht worden ist, in welches das Stielteil 207 des Anschlußelementes 200 hineingesetzt werden kann. Das erste Anschlußelement wird also intramedullär verankert. Ein wichtiger Nebenaspekt ist im übrigen, daß das Stielteil außen ebenso wie die plattenförmige Basis zum Darmbeinstumpf hinweisend eine offenmaschige dreidimensionale Raumnetzstruktur aus Metall aufweisen kann, in welche Knochenmaterial zur dauerhaften Fixation des Implantates einwachsen kann.

Das Stielteil durchsetzt die Basis 202 in einem Langloch 212 (Figur 8). Unterhalb der Basis 202 schließt sich dem Stielteil 207 der Zapfen 201 an.

Wesentlich ist, daß der Zapfen 201 von dorsal nach ventral gesehen in dem Winkel γ geneigt ist, wobei der Winkel γ im Bereich zwischen 25° und 45° liegt und vorzugsweise 35° beträgt (Figur 6). Der Zapfen ist darüber hinaus von medial nach lateral in dem Winkel δ geneigt, der in einem Bereich von 0° bis 10° liegt und vorzugsweise 5° beträgt (Figur 7).

Erkennbar aus Figur 6 ist im übrigen noch der Sicherungssplint 208, der - wie in Figur 1 angedeutet - nach Erreichen der Endstellung in der Aufnahmebohrung unter Federkraft in die Tangentialnut 106 springt.

Erkennbar sind im übrigen die Rotationssicherungsstifte 210, die an der Basis des Zapfens angeordnet sind und nach Herstellung der Verbindung zum Grundkörper 100 in die Bohrungen 110 im Grundkörper greifen.

Im gezeigten Ausführungsbeispiel ist im übrigen das Stielteil 207 von der Achse des Zapfens 201 um einige Millimeter nach dorsal versetzt angeordnet. Diese Versetzung kann in der Ausführung zwischen 5 bis 20 mm betragen. Diese versetzte Anordnung ist aus anatomischen Gründen zweckmäßig, da das Stielteil 207 dann in einem intramedullären Raum gesetzt werden kann, der hierfür genügend Platz bietet.

Im gezeigten Ausführungsbeispiel ist im übrigen eine mechanische Verstelleinheit 206 in Form einer Gewindespindel vorgesehen, mit Hilfe derer das Stielteil in dem Langloch 212 in der Basis 202 hin- und hergefahren werden kann, solange, bis intraoperativ die optimale Stellung ermittelt worden ist.

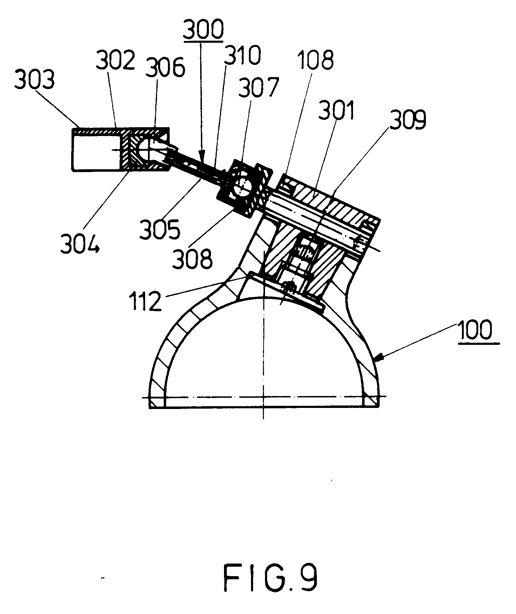

Aus Figur 9 wird im übrigen die Verbindung zwischen dem Grundkörper 100 und dem zapfenförmigen Verbindungselement 301 ersichtlich. Dieses ist im dargestellten Ausführungsbeispiel - wie im übrigen auch der Zapfen 201 des ersten Anschlußelementes 200 als konischer Steckzapfen ausgebildet, der in die entsprechend als konische Klemmhülsen ausgebildeten Aufnahmebohrungen setzbar ist. Angedeutet ist im übrigen, wie eine Sicherungsschraube von der Innenseite der Kalotte in das zapfenförmige Verbindungselement 301 greift, wobei sich der Schraubenkopf gegen die Ringschulter 112 legt.

Kern der Figur 9 aber ist das zweite Anschlußelement 300 zur Verbindung des Schambeinstumpfes 410 mit dem Grundkörper 100. Dessen schambeinseitiges Anschlußstück 302 weist eine am Schambeinstumpf zu befestigende Lasche 303 auf. Die Befestigung erfolgt beispielsweise über Knochenschrauben (nicht dargestellt). Dieser Lasche angeformt ist ein Kugelkäfig 304, in dem eine Kugel 306 des Überbrückungselementes 305 gelagert ist. Das Überbrückungselement besteht im dargestellten Ausführungsbeispiel aus einer in seiner Länge verstellbaren Gewindehülse 310, die an dem anderen Ende eine weitere Kugel 307 trägt, welche in einem weiteren Kugelkäfig 308 gelagert ist, an dem rückseitig ein Fixationsstab 309 befestigt ist, der mit dem zapfenförmigen Verbindungselement 301 verschraubt ist. Der Fixationsstab 309 greift im übrigen durch die Tangentialnut 108, wie bereits oben erwähnt.

Diese eingangs als hantelförmig bezeichnete Ausführungsform für das Überbrückungselement sorgt für einen relativ festen Schluß zwischen Schambeinstumpf und Grundkörper 100. Die Kugellagerung gestattet aber die Ausübung von Mikrobewegungen gegeneinander.

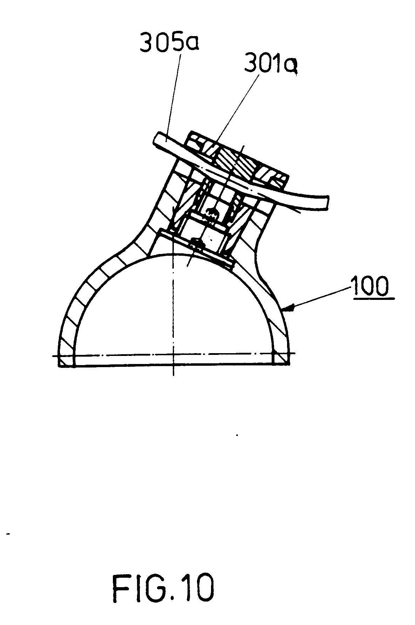

Einen etwas lockereren Verbund zwischen Schambeinstumpf und Grundkörper bietet eine andere Ausführungsform des zweiten Anschlußelementes, welches zwar auch über die Teile 302, 303, 304 und 306 des ersten Ausführungsbeispiels verfügt. Allerdings ist hier keine Gewindehülse 305 mit einem weiteren Kugelkäfig 308 vorgesehen, sondern vielmehr ein Drahtseil 305a (Figur 10), welches in ein anderes zapfenförmiges Verbindungselement 301a greift und dort verspannt ist. Die Verspannung kann man sich bildlich vorstellen wie die Befestigung eines Bowdenzuges.

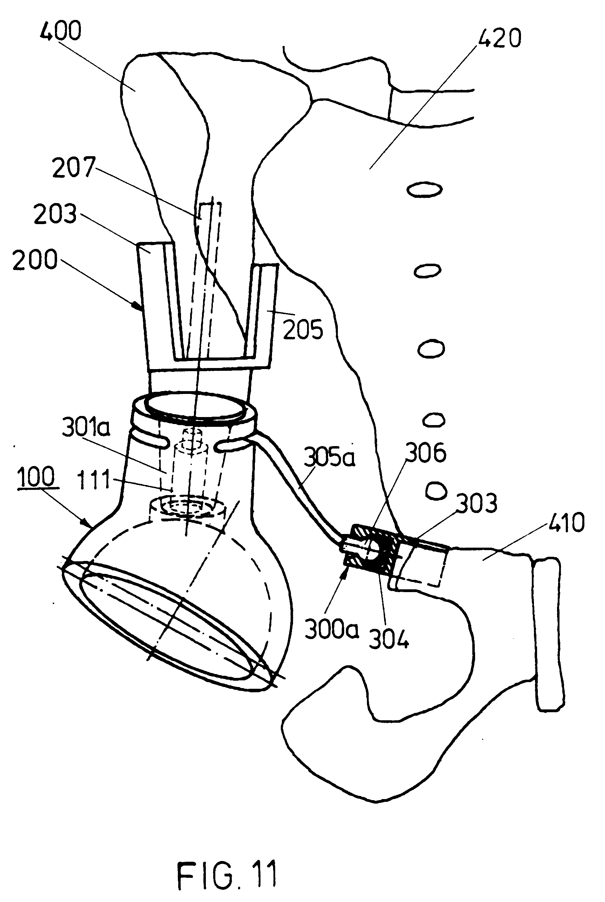

Aus Figur 11 ist eine vollständige, dreiteilige Beckenteilendoprothese ersichtlich, welche mit der zweiten Ausführungsform des zweiten Anschlußelementes 300a ausgestattet ist. Hierbei zeigt Figur 11 eine Ansicht der Endoprothese in Richtung des Pfeiles XI in Figur 1, allerdings ergänzt um das zweite Anschlußelement 300a.

Die erfindungsgemäße Endoprothese zeichnet sich aus durch die Möglichkeit, daß die einzelnen Elemente auch noch intraoperativ ausgetauscht werden können. Deren Einbaulage kann auch noch während der Implantation mit hinreichender Genauigkeit eingestellt werden. Sie läßt sich je nach den individuellen Patientenerfordernissen aus einem Set erstellen, ohne daß für jeden Patienten nun individuelle Gußformen erstellt werden müssen, um ein individuelles Implantat in Metall gießen zu können.

## Patentansprüche

1. Beckenteilendoprothese zum Ersatz eines Beckenteils im Bereich des Hüftgelenks mit einem Grundkörper (100), der eine kalottenförmige Aussparung (101) zur Aufnahme einer künstlichen Hüftgelenkspfanne sowie einen Ansatz (102) aufweist, in welchem mindestens eine Aufnahmebohrung (103, 104) ausgebildet ist und einem ersten Anschlußelement (200) zur Herstellung einer Verbindung des Grundkörpers (100) mit dem Darmbeinbereich (400) des Restbeckens, wobei die Verbindung über einen selbsthemmenden Sitz eines Zapfens (201) des ersten Anschlußelementes (200) in der wenigstens einen Aufnahmebohrung (103, 104) im Ansatz (102) des Grundkörpers (100) herstellbar ist, wobei die Längsachse der mindestens einen Aufnahmebohrung (103, 104) mit der Polachse (105) der Aussparung (101) einen Winkel β im Bereich von 20° < β < 30°, vorzugsweise β = 25°, einschließt,
dadurch gekennzeichnet, daß der Grundkörper (100) und der Ansatz (102) eine bauliche Einheit bilden und daß der Zapfen (201) von dorsal nach ventral gesehen in einem Winkel γ im Bereich 25° < γ < 45°, vorzugsweise γ = 35°, und von medial nach lateral in einem Winkel δ = 5°, geneigt ist.

2. Beckenteilendoprothese nach Anspruch 1, bei der im Ansatz (102) zwei Aufnahmebohrungen (103, 104) ausgebildet sind, deren Längsachsen einen Winkel α im Bereich von 40°< α <60°, vorzugsweise α = 50° miteinander und jeweils den Winkel β im Bereich von 20°< β <30°, vorzugsweise β = 25° mit der Polachse (05) der Aussparung (107) einschließen.

3. Beckenteilendoprothese nach Anspruch 2, bei der mit einem zweiten Anschlußelement (300) zur Herstellung einer Verbindung des Grundkörpers (100) mit dem Schambeinbereich (410) des Restbeckens eine Verbindung über einen selbsthemmenden Sitz eines zapfenförmigen Verbindungslementes (301) in der anderen Aufnahmebohrung (103, 104) im Ansatz (102) des Grundkörpers (100) herstellbar ist.

4. Beckenteilendoprothese nach Anspruch 2 oder 3, bei der die beiden Aufnahmebohrungen (103, 104) gleiche Größe und gleiche Form aufweisen und die Außenkonturen des zapfenförmigen Verbindungselementes (301) des zweiten Außenelementes (300) jeden des Zapfen (201) des ersten Anschlußelementes (200) entsprechen.

5. Beckenteilendoprothese nach einem der Ansprüche 2 bis 4, bei der die Aufnahmebohrungen (103, 104) als konische Klemmhülsen und der Zapfen (201) und das zapfenförmige Verbindungselement (301) als konische Steckzapfen ausgebildet sind.

6. Beckenteilendoprothese nach einem der Ansprüche 1 bis 5, bei der die Aufnahmebohrungen (103, 104) im Ansatz (102) bis in die Innenkalotte des Grundkörpers (100) reichen, so daß sie auch von der Innenseite des Grundkörpers zugänglich sind.

7. Beckenteilendoprothese nach einem der Ansprüche 1 bis 6, bei der das erste Anschlußelement (200) eine Basis (202) zur Anlage an dem Darmbeinbereich (400) aufweist, von der lateral zwei Anschlagslaschen (203, 204) und medial eine Anschlagslasche (205) abragt, zwischen denen der Restknochen anliegt und bei der ein mittels einer mechanischen Verstelleinheit (206) entland der Basis (202) verfahrbares und diese durch ein Langloch (22) durchsetzendes, in eine im Restbecken intramedullär anzubringenden Nut zu setzendes Stielteil (207) vorgesehen ist, an welchem in Richtung auf den Grundkörper (100) der Zapfen (201) angeformt ist.

8. Beckenteilendoprothese nach Anspruch 7, bei der das Stielteil (207) des ersten Anschlußelementes (200) von der Achse des Zapfens (201) um 5 mm bis 20 mm nach dorsal versetzt angeordnet ist.

9. Beckenteilendoprothese nach einem der Ansprüche 1 bis 8, bei der außen am Ansatz (102) beiderseitig beider Aufnahmebohrungen (103, 104) jeweils zwei tangential verlaufende Nuten (106, 107; 108, 109) vorgesehen sind, welche die Aufnahmebohrungen bereichsweise tangential durchbrechen.

10. Beckenteilendoprothese nach Anspruch 9, bei der der selbsthemmende Sitz des Zapfens (201) in einer der Aufnahmebohrungen (102, 103) zusätzlich gesichert wird durch Einrasten eines Sicherungssplintes (208) des Zapfens (201) in die jeweilige Nut (106, 107; 108, 109) im Ansatz (102).

11. Beckenteilendoprothese nach einem der Ansprüche 1 bis 10, bei der das zweite Anschlußelement (300) ein schambeinseitiges Anschlußstück (302), aufweisend eine am Schambeinstumpf befestigbare Lasche (303) sowie einen Kugelkäfig (304), sowie ein Überbrückungselement (305, 305a) aufweist, dessen eines Ende als im Kugelkäfig (304) gelagerte Kugel (306) ausgebildet ist und dessen anderes Ende mit dem zapfenförmigen Verbindungselement (301, 301a) verbindbar ist.

12. Beckenteilendoprothese nach Anspruch 11, bei der das jeweilige Überbrückungselement durch eine der jeweiligen Nuten (106, 107; 108, 109) in das zapfenförmige Verbindungselement (301, 301a) greift und dort mit ihm verschraubt ist.

13. Beckenteilendoprothese nach Anspruch 11 oder 12, bei dem das Überbrückungselement (305) besteht aus einer Gewindehülse (310), die an ihrem dem Grundkörper (100) zugewandten Ende eine weitere Kugel (307) trägt, die in einen Kugelkäfig (308) greift, an dem rückseitig ein Fixationsstab (309 befestigt ist, der mit dem zapfenförmigen Verbindungselement (301) verschraubbar ist.

14. Beckenteilendoprothese nach Anspruch 11 oder 12, bei der das Überbrückungselement (305a) aus einem Drahtseil besteht, welches im Inneren des zapfenförmigen Verbindungselementes (301a) verschraubbar ist.

## Claims

1. Partial pelvic endoprosthesis to replace a pelvis part in the region of the hip joint, having a main body (100), which has a dome-shaped recess (101) to receive an artificial hip joint acetabulum and an attachment (102), in which is formed at least one receiver bore (103, 104) and a first connection element (200) to produce a connection of the main body (100) with the ilium region (400) of the remaining pelvis, wherein the connection can be made via a self-locking seat of a journal (201) of the first connection element (200) in at least one receiver bore (103, 104) in the attachment (102) of the main body (100), wherein the longitudinal axis of at least one receiver bore (103, 104) encloses an angle β in the region of 20° < β < 30°, preferably β = 25°, with the polar axis (105) of the recess (101), characterised in that the main body (100) and the attachment (102) form a constructional unit, and in that the journal (201), seen dorsally to ventrally, is inclined at an angle γ in the region 25° < γ < 45°, preferably γ = 35°, and medially to laterally at an angle δ = 5°.

2. Partial pelvic endoprosthesis according to claim 1, in which two receiver bores (103, 104), the longitudinal axes of which enclose an angle α in the region of 40° < α < 60°, preferably α = 50°, with one another, and in each case the angle β in the region of 20° < β < 30°, preferably β = 25°, with the polar axis (105) of the recess (107), are formed in the attachment (102).

3. Partial pelvic endoprosthesis according to claim 2, in which using a second connection element (300) to produce a connection for the main body (100) with the pubic bone region (410) of the remaining pelvis, a connection can be made via a self-locking seat of a journal-like connecting element (301) in the other receiver bore (103, 104) in the attachment (102) of the main body (100).

4. Partial pelvic endoprosthesis according to claim 2 or 3, in which the two receiver bores (103, 104) have the same size and the same shape, and the outer contours of the journal-like connecting element (301) of the second outer element (300) correspond to those of the journal (201) of the first connection element (200).

5. Partial pelvic endoprosthesis according to one of claims 2 to 4, in which the receiver bores (103, 104) are designed as conical split taper sockets, and the journal (201) and the journal-like connecting element (301) as conical plug journals.

6. Partial pelvic endoprosthesis according to one of claims 1 to 5, in which the receiver bores (103, 104) in the attachment (102) extend to the inner dome of the main body (100), so that they are also accessible from the inner side of the main body.

7. Partial pelvic endoprosthesis according to one of claims 1 to 6, in which the first connection element (200) has a base (202) for resting against the ilium region (400), from which project laterally two stop brackets (203, 204) and medially one stop bracket (205), between which the remaining bone rests, and in which a stem part (207) to be placed in a groove to be introduced intramedullarly in the remaining pelvis and which can be moved by means of a mechanical adjusting unit (206) along the base (202) and penetrating the latter through an elongated hole (22), is provided, on which stem part (207) the journal (201) is moulded in the direction of the main body (100).

8. Partial pelvic endoprosthesis according to claim 7, in which the stem part (207) of the first connection element (200) is arranged to be dorsally displaced from the axis of the journal (201) by 5 mm to 20 mm.

9. Partial pelvic endoprosthesis according to one of claims 1 to 8, in which in each case two tangentially extending grooves (106, 107; 108, 109) are provided externally to the attachment (102) on both sides of both receiver bores (103, 104) and breach the receiver bores tangentially in some regions.

10. Partial pelvic endoprosthesis according to claim 9, in which the self-locking seat of the journal (201) is additionally secured in one of the receiver bores (102, 103) by locking a security split pin (208) of the journal (201) in the particular groove (106, 107; 108, 109) in the attachment (102).

11. Partial pelvic endoprosthesis according to one of claims 1 to 10, in which the second connection element (300) has a pubic bone-side connection piece (302) having a bracket (303) which can be attached to the pubic bone stump and a ball cage (304) as well as a bridging element (305, 305a), one end of which is designed as a ball (306) mounted in the ball cage (304) and the other end of which can be connected to the journal-like connecting element (301, 301a).

12. Partial pelvic endoprosthesis according to claim 11, in which the particular bridging element engages through one of the particular grooves (106, 107; 108, 109) into the journal-like connecting element (301, 301a) and is screwed there to it.

13. Partial pelvic endoprosthesis according to claim 11 or 12, in which the bridging element (305) consists of a threaded sleeve (310), which supports a further ball (307) at its end facing the main body (100), which ball (307) engages in a ball cage (308) to which is attached a fixing rod (309) on the rear side, which can be screwed to the journal-like connecting element (301).

14. Partial pelvic endoprosthesis according to claim 11 or 12, in which the bridging element (305a) consists of a wire rope which can be screwed in the interior of the journal-like connecting element (301a).

## Revendications

1. Endoprothèse partielle du bassin destinée à remplacer une partie du bassin au niveau de l'articulation de la hanche, comportant un corps de base (100), qui possède un évidement en forme de calotte (101) servant à loger un coussinet artificiel de l'articulation de la hanche ainsi qu'une partie saillante (102), dans lequel au moins un perçage de logement (103, 104) est formé, et un premier élément de raccordement (200) pour établir une liaison entre le corps de base (100) et la partie iliaque (400) du reste du bassin, la liaison pouvant être établie par l'intermédiaire d'un montage autobloquant d'un téton (201) du premier élément de raccordement (200) dans au moins un perçage de logement (103, 104) situé dans la partie saillante (102) du corps de base (100), l'axe longitudinal du au moins un perçage de logement (103, 104) faisant avec l'axe polaire (105) de l'évidement (101) un angle β situé dans la gamme 20° < β < 30° et égal de préférence à β = 25°,
caractérisé en ce que le corps de base (100) et la partie saillante (102) forment une unité de construction, et que le téton (201) est incliné, dans le sens allant du côté dorsal vers le côté ventral, sous un angle γ dans la gamme 25° < γ < 45°, de préférence avec γ = 35°, et du côté médian vers le côté latéral, sous un angle δ = 5°.

2. Endoprothèse partielle du bassin selon la revendication 1, dans laquelle dans la partie saillante (102) sont formés deux perçages de logement (103, 104), dont les axes font entre eux un angle α dans la gamme 40° < α < 60°, avec de préférence α = 50°, et font avec l'axe polaire (05) de l'évidement (107) respectivement l'angle β, dans la gamme 20° < β < 30°, avec de préférence β = 25°.

3. Endoprothèse partielle du bassin selon la revendication 2, dans laquelle une liaison peut être établie avec un second élément de raccordement (300) servant à établir une liaison du corps de base (100) avec la partie pubienne (410) du reste du bassin, au moyen d'un montage autobloquant d'un élément de liaison en forme de téton (301) dans l'autre perçage de logement (103, 104) dans la partie saillante (102) du corps de base (100).

4. Endoprothèse partielle du bassin selon la revendication 2 ou 3, dans laquelle les deux perçages de logement (103, 104) possèdent la même taille et la même forme, et les contours extérieurs de l'élément de liaison en forme de téton (300) du second élément extérieur (300) correspondent à ceux du téton (201) du premier élément de raccordement (200).

5. Endoprothèse partielle du bassin selon l'une des revendications 2 à 4, dans laquelle les perçages de logement (103, 104) sont agencés sous la forme de douilles coniques de serrage et le téton (201) de l'élément de liaison en forme de téton (301) sont réalisés sous la forme de tétons enfichables coniques.

6. Endoprothèse partielle du bassin selon l'une des revendications 1 à 5, dans laquelle les perçages de logement (103, 104) formés dans la partie saillante (102) s'étendent jusque dans la calotte intérieure du corps de base (100), de sorte qu'ils sont accessibles également à partir du côté intérieur du corps de base.

7. Endoprothèse partielle du bassin selon l'une des revendications 1 à 6, dans laquelle le premier élément de raccordement (200) comporte une base (202) destinée à s'appliquer contre la partie iliaque (400) du bassin, à partir de laquelle s'étendent latéralement deux pattes de butée (203, 204) et, en position médiane, une patte de butée (205), pattes entre lesquelles est situé l'os résiduel, et dans laquelle est prévu un élément en forme de tige (207), qui est déplaçable au moyen d'une unité de réglage mécanique (206) le long de la base (202) et traverse cette dernière au niveau d'un trou allongé (22) et doit être placée dans une rainure, qui doit être aménagée de façon intramédulaire dans le reste du bassin, et que le téton (201) est formé sur cette partie en forme de tige, de manière à être dirigé vers le corps de base (100).

8. Endoprothèse partielle du bassin selon la revendication 7, dans laquelle l'élément en forme de tige (207) du premier élément de raccordement (200) est disposé en étant décalé sur ledit côté dorsal de 5 mm à 20 mm par rapport à l'axe du téton (201).

9. Endoprothèse partielle du bassin selon l'une des revendications 1 à 8, dans laquelle sur la partie saillante (102) sont aménagées extérieurement, et ce des deux côtés des deux perçages de logement (103, 104), respectivement deux rainures tangentielles (106, 107 ; 108, 109), qui traversent tangentiellement, par endroits, les perçages de logement.

10. Endoprothèse partielle du bassin selon la revendication 9, dans laquelle le montage autobloquant du téton (201) dans l'un des perçages de logement (102, 103) est bloqué d'une manière supplémentaire au moyen de l'encliquetage d'une goupille de fixation (208) du téton (201) dans la rainure respective (106, 107 ; 108, 109) formée dans la partie saillante (102).

11. Endoprothèse partielle du bassin selon l'une des revendications 1 à 10, dans laquelle le second élément de raccordement (300) comporte un élément de raccordement pubien (302), qui comporte une patte (303) pouvant être fixée sur le tronc iliaque ainsi qu'une cage à bille (304), ainsi qu'un élément de pontage (305, 305a), dont une extrémité est agencée sous la forme d'une bille (306) montée dans la cage à bille (304) et dont l'autre extrémité peut être reliée à l'élément de liaison en forme de téton (301, 301a).

12. Endoprothèse partielle du bassin selon la revendication 11, dans laquelle l'élément de pontage respectif s'engage à travers l'une des rainures respectives (106, 107 ; 108, 109) dans l'un des éléments de liaison en forme de tétons (301, 301a) et y est fixé par vissage.

13. Endoprothèse partielle du bassin selon la revendication 11 ou 12, dans laquelle l'élément de pontage (305) est constitué par une douille de filetée (310), qui porte, sur son extrémité tournée vers le corps de base (100), une autre bille (307) qui s'engage dans une cage à bille (308), sur le côté arrière de laquelle est fixée une barre de fixation (309), qui est vissable sur l'élément de liaison en forme de téton (301).

14. Endoprothèse partielle du bassin selon les revendications 11 ou 12, dans laquelle l'élément de pontage (305a) est constitué par un câble métallique, qui peut être vissé à l'intérieur de l'élément de liaison en forme de téton (301a).
